# EUROPÄISCHE PATENTANMELDUNG

(11) **EP 0 624 344 A2**
(43) Veröffentlichungstag der Anmeldung: **17.11.1994**
(21) Anmeldenummer: 93111000.1
(22) Anmeldetag: 09.07.1993
(51) Int. Cl.: A61B 17/22, A61B 17/36

(54) **Diathermiehandstück mit endoskopischer Sonde**

(30) Priorität: 13.04.1993 DE 9305428 U; 12.05.1993 DE 9307183 U
(71) Anmelder: SÖRING MEDIZIN TECHNIK GmbH, D-25712 Quickborn (DE)
(72) Erfinder: Söring, Holger, D-2085 Quickborn (DE)

(57) **Zusammenfassung**

Die vorliegende Erfindung betrifft ein Diathermiehandstück mit endoskopischer Sonde für den Einsatz bei chirurgischen Einsätzen insbesondere bei lapraskopischen Ultraschalldissektionen, bei denen eingriffsbedingt auftretende Blutungen durch Verschweißen von Blutgefäßen gestoppt werden müssen, wobei der wesentliche Vorteil dieser Lösung darin gesehen wird, Coagulation und Dissektion mit einem Gerät möglich zu machen, d.h. daß die die Gaszuleitung **19** und den Absaugkanal **6** aufnehmende Sonotrode **8** elektrisch leitend aus gestaltet ist. Das Diathermiehandstück kann mit einer endokopischen Sonde verbunden sein, die die Einsatzmöglichkeiten dieses Gerätes noch erheblich verbessern.

## Beschreibung

Die vorliegende Erfindung betrifft ein Diathermiehandstück für den Einsatz bei chirurgischen Eingriffen. Mit Hilfe derartiger Instrumente werden bekanntermaßen Krebstumore unter Verwendung von Ultraschallschwingungen operativ entfernt. Diese Ultraschall-Schwingungen werden in einem Griffbereich des Handstückes erzeugt und auf eine das Krebstumorgewebe abtragende Sonde übertragen und im Bereich der dem Operationsfeld zugewandten Sondenspitze mündet eine Spülmittelzuleitung und eine Absaugleitung.

Derartige Handstücke haben sich bei der operativen Beseitigung von Krebstumoren bewährt. Das Handstück ist Teil eines Ultraschall-Gewebezertrümmerers. Das Funktionsprinzip besteht darin, daß über sogenannte Transducer, die im Griffbereich des Handstückes untergebracht sind, eine Sonde in hochfrequente longitudinale Schwingungen versetzt wird (ca. 24 kHz.). Die dem Griffbereich abgewandte Sondenspitze führt longitudinale Schwingungen aus, die Krebsgewebe zerstören, welches wesentlich empfindlicher gegen mechanische Beanspruchungen ist als gesundes Gewebe. Durch die Einwirkung der Schwingungen im Bereich der Spitze der Sonde wird das damit in Berührung kommende Tumorgewebe aufgelöst und mit Hilfe einer Spülflüssigkeit, die im Bereich der Sondenspitze über eine Spülmittelzuleitung herangeführt wird, über eine Absaugleitung abgesaugt.

Bei derartigen operativen Eingriffen können bedingt durch Gefäßverletzungen starke Blutungen auftreten, die schnellstmöglich gestoppt werden müssen. Überlicherweise werden derartige Blutungen mit Hilfe zusätzlicher Werkzeuge wie Klemmen, Heizdrahtelektroden etc. gestoppt.

Ein Handstück mit im Gerät angeordneter Spülmittelzuleitung und integrierter Absaugleitung ist z.B. in dem DE-GM Nr. 89 14 513 beschrieben.
Auch bei mit diesem Gerät durchgeführten Operationen ist es bei Auftreten starker Blutungen nur möglich, diese beispielsweise mittels der vorbeschriebenen Zusatzgeräte zu stoppen.

Bei offenen Operationsfeldern ist es inzwischen eine bekannte Koagulationstechnik mit Hilfe von Mikro-Elektro-Schutzgas-Schweißgeräten Blutungen durch Verschweißen der Kapillargefäße zu stoppen. Derartige Geräte sind z.B. ausführlich in der DE-OS 37 10 489 beschrieben. So soll es die in dieser Patentanmeldung beschriebene Vorrichtung, bei der zur elektrochirurgischen Koagulation elektrische Energie in Lichtbögen zu einem von Blut durchströmten Gewebe in einem Lebewesen geleitet wird, ermöglichen, daß durch Leiten eines insgesamt laminaren Strahls ionisierbaren Gases Blut von der Oberfläche eines Gewebes entfernt werden kann, wobei durch das Leiten der elektrischen Energie in Lichtbögen ein Schorf mit vorbestimmten Eigenschaften erzeugt werden soll.
Das hierfür erforderliche stiftartige Handstück, das durch den Chirurgen während der chirurgischen Prozedur manipulierbar ist, weist eine Düse zum Erzeugen des Gasstrahles und eine Einrichtung zum Übertragen der elektrischen Energie mittels einer Elektrode in dem Casstrahl auf, wobei eine Schnur das Handstück mit einer Gasversorgungseinrichtung und einem Generator verbindet. Diese Schnur ist so gestaltet, daß über ihre Länge mehrere um einen elektrischen Leiter angeordnete Gasleithohlräume angeordnet sind, die sich insgesamt parallel zu dem elektrischen Leiter erstrecken.
Das stiftartige Handstück besteht aus einem Griff, der mit der Schnur verbunden ist, einer Düsen- und Elektrodenhalteeinrichtung, mit der die Düse und die Elektrode integral verbunden sind und einer Kupplungseinrichtung, die mit dem Griff verbunden ist, zum lösbaren Verbinden der Düsen- und Elektrodenhalteeinrichtung mit dem Haltestück, wobei die Kupplungseinrichtung außerdem die Elektrode mit dem elektrischen Leiter der Schnur elektrisch verbindet und außerdem Gas aus dem Gasleitkanal in die Düse leitet.

Eine Weiterbildung eines derartigen Mikro-Schweißgerätes ist in dem DE-Gebrauchsmuster Nr. 91 04 559 beschrieben.
Die in diesem Gebrauchsmuster beschriebene Lösung stellt gegenüber dem in der DE-OS 37 10 489 beschriebenen Gerät eine erhebliche Vereinfachung dar.

Alle diese bekannten Verfahren zur Inertgas-Koagulation nutzen einen zentralen elektrischen Leiter im Spülgasstrom für die Zufuhr der elektrischen Energie zur Ionisation des Gases an der Austrittsstelle.

Da die zentrale Elektrode über weite Strecken innerhalb des Strömungsraumes (Rohr) nicht gegenüber der Wand fixiert ist, kommt es, je nach Stärke des Leiters, zu mehr oder weniger ausgeprägten undefinierten Ortsveränderungen und Biegeschwingungen. Damit wird die Form des Strömungsquerschnittes ständig verändert. Das ist die Ursache für die Entstehung von Turbulenzen im Gasstrom.

Die Rückkopplung dieser Turbulenzen auf die schwingungsfähige Elektrode bewirkt wiederum eine Verstärkung der Schwingungen und damit ein "Aufschaukeln" der Turbulenzen bis hin zu kurzzeitigen Resonanzen. Diese äußern sich in den bei diesen Verfahren gehäuft auftretenden singenden, bzw. prasselnden Geräuschen. Auch die elektrischen Instabilitäten des gezündeten Plasmastrahles haben ihre Ursache in der Ausbildung der auf Resonanzen beruhenden stehenden Wellen, die sich in einer Schichtung des Plasmas in helle und dunklere Bereiche äußern.
Durch diese verfahrensbedingten, vorprogrammierten Verwirbelungen kommt es zum Eintrag von Luftsauerstoff in den Spülgasstrahl und damit zu den Oxidationsvorgängen auf der zu koagulierenden Gewebeoberfläche, die man mit der Wahl eines Inertgases als Spülgas eigentlich vermeiden will. Ein Indiz für den Lufteintrag ist die charakteristische Verfärbung des Plasmastrahles.

Die Zündung des Plasmastrahles durch Ionisierung des Gasstromes von dessen Zentrum ausgehend, bewirkt aufgrund thermodynamischer Wechselwirkungen (Dichte- und Temperaturunterschiede über den Gasstromquerschnitt) eine zusätzliche Aufweitung des Plasmastrahles. Dieser erreicht schließlich einen Wirkungsquerschnitt bis zu mehreren Quadratmillimetern auf der zu koagulierenden Gewebeoberfläche.

Die Ausweitung wird durch elektromagnetische Wirbelfelder innerhalb des hochionisierten Kernbereiches noch verstärkt.
Damit ist ein punktgenaues Arbeiten -wie es beispielsweise die Mikro- und Neurochirurgie fordert- nicht möglich.

Der Erfindung liegt daher die Aufgabe zugrunde, ein Handstück der eingangs erwähnten Art zu schaffen, mit dessen Hilfe nicht nur eine sichere und gute Abtragung und Entfernung von Krebstumorgewebe u.a. aus dem Operationsfeld möglich ist, sondern auch während des Eingriffs auftretende Blutungen durch Verschweißen von Gefäßen gestoppt werden können.

Die Lösung dieser Aufgabe besteht darin, daß im zentralen Bereich des Handstückes eine Gaszuführung angeordnet ist und daß die als Gaszuleitung und Absaugkanal dienende Sonotrode elektrisch leitend ausgestaltet ist.

In einer besonderen Ausgestaltung kann das Handstück mit einer - hier nicht dargestellten- Kupplungseinrichtung versehen sein, welche für eine gasdichte und elektrisch sichere Verbindung zwischen Handstück und einer endoskopischen Sonde sorgt. Eine derartige Sonde ist zweckmäßigerweise mehrschichtig aufgebaut, wobei ein elektrisch leitfähiges Material durch einen isolierenden Überzug den Benutzer der Sonde sowie den Patienten schützt.

Weitere zweckdienliche Ausgestaltungen eines derartigen Diathermie-Handstückes sind in den Unteransprüchen erläutert.

Ein Ausführungsbeispiel der Erfindung ist in der folgenden Beschreibung im Zusammenhang mit den Zeichnungen näher beschrieben.

Es zeigt:

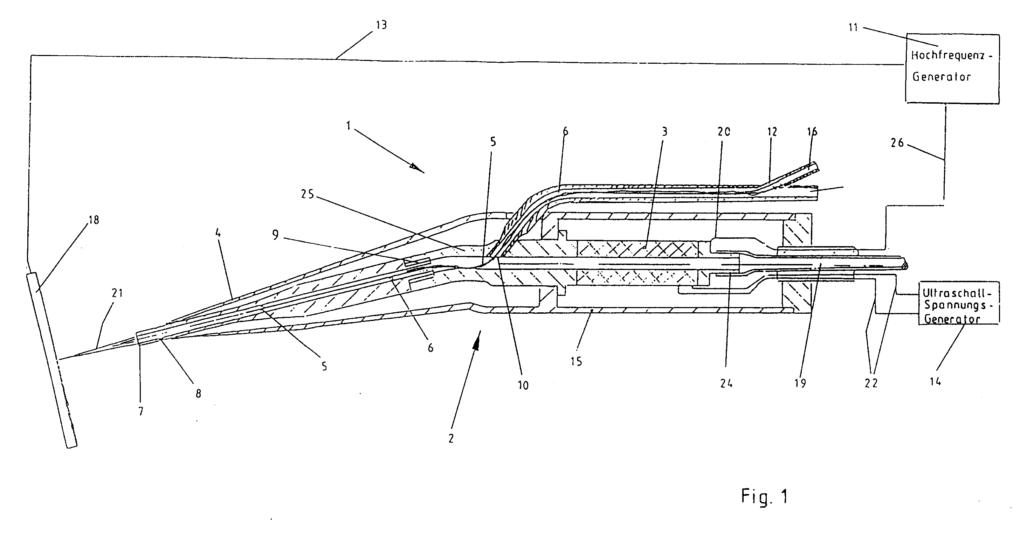

- Fig. 1: einen Längsschnitt durch ein Diathermiehandstück;

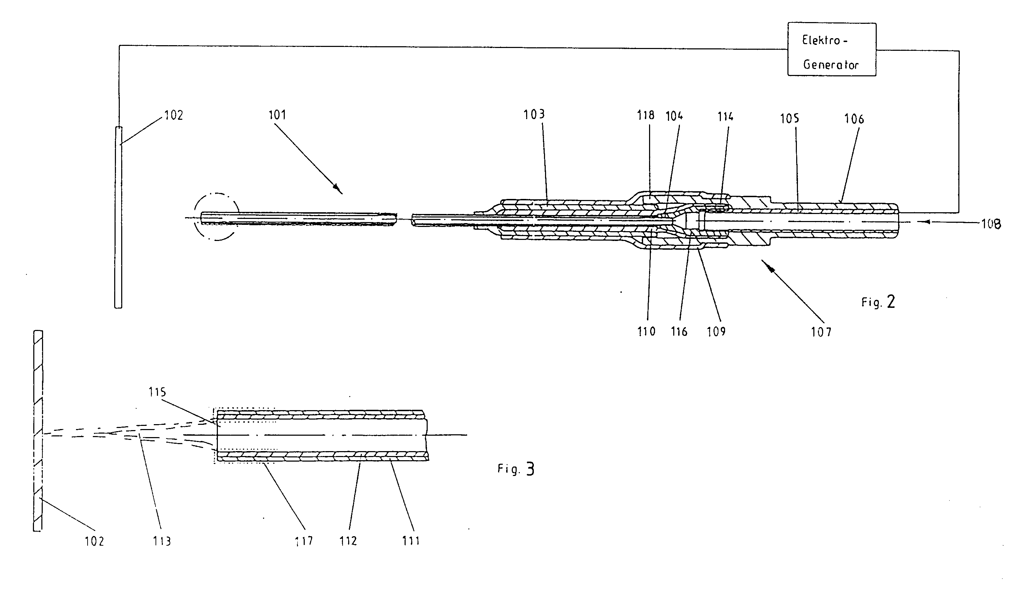

- Fig. 2: einen Längsschnitt durch eine endoskopische Sonde;
- Fig. 3: eine vergrößerte Darstellung des Austrittsbereiches der Sonde
Ein Handstück **1** besteht aus einer mit einem Griffbereich **2** versehenen Griffhülse **15**, in der Transducer **3** zur Erzeugung von Ultraschallschwingungen vorgesehen sind und einem Verbidungsteil **25** , welches im Bereich der Sonotrodenaufnahme **9** die Sonotrode **8** trägt und außerdem die Spülmittelleitung **5** und die Absaugleitung **6** sowie die Gasführung **19** und die Zuleitungen **22** und **26** aufnimmt.

Die Sonotrode **8** besteht vorzugsweise aus Titan, es sind aber auch andere Materialien denkbar. In dieser als Rohr gestalteten Sonotrode ist eine Spülmittelzuleitung **5** gelagert, die durch das Verbindungsteil **25** im Mündungsbereich **10** in die Absaugleitung **6** geführt ist, aus dieser in einem Abzweigstück **12** austritt und in dem Anschlußteil **16** für die Spülmittelzuleitung **5** endet. Über die Absaugleitung **6** können Flüssigkeit und Gewebereste vom Operationsfeld abgesaugt werden. Die Absaugleitung führt durch die Sonotrode **8**, das Verbindungsteil **25**, die Absaugleitung **6** zum Anschluß **17** für die Absaugleitung. Mittels hier nicht dargestellter vorzugsweise mit dem Fuß zu betätigender Schalter kann der Chirurg die Spül- bzw. die Absaugeinrichtung ein- bzw. ausschalten.

In dem Handstück ist ferner eine Gaszuführungsleitung **19** an dem Adapter **24** für diese Leitung befestigt. Außerdem ist eine Verbindungsleitung **26** vom HF-Generator **11** zum Hochfrequenzanschluß **20** innerhalb des Handstückes **1** vorgesehen, wobei diese Zuleitung mittels eines hier ebenfalls nicht dargestellten Schalters vom Chirurgen unterbrochen werden kann.

Im Falle einer während einer Operation notwendigen Schutzgaskoagulation wird zunächst die Gaszuführung **19** freigegeben und anschließend wird durch Einschalten der HF-Spannung der zum Koagulieren notwendige Plasmastrahl ausgelöst. Um ein sicheres "Zünden" des Plasmastrahles zu gewährleisten, ist für einen guten Kontakt zwischen Patient und Gegenpolplatte **18** zu sorgen, die über eine Zuleitung **13** mit dem Hochfrequenzgenerator **11** verbunden ist.

Nach Abschluß des Koagulierens kann durch einfaches Umschalten die Ultraschalldissektion fortgeführt werden. Der Ultraschallspannungs-Generator ist mittels Zuleitungen **22** mit den Ultraschall-Anschlüssen **23** des Transducer **3** verbunden.

Die Austrittsöffnung **7** der Sonotrode **8** kann im Innen- und im Stirnbereich mit einem keramischen Überzug versehen sein, wodurch ein Verschmutzen der Sonotrodenspitze verhindert wird. Die mittels einer hier nicht dargestellten Kupplungseinrichtung mit dem Handstück verbundene endoskopische Sonde **101** besteht aus einem flexiblen Rohr **(111,112),** einem Sondenkopf **116** und einem Kupplungsteil **107**.
Das flexible Rohr **(111,112)** besteht auf seiner Innenseite aus einem elektrisch leitenden Material, z.B. aus einer nach Art eines Bowdenzuges hergestellten Spirale oder aus einem Metallgewebe **111** und einem den elektrisch leitenden Innenbereich gas- und HF-dicht isolierenden Schutzschlauch **(111)**. Dieser Schutzschlauch **(111)** kann aus unterschiedlichen Materialien bestehen; vorzugsweise wird ein PTFE o.ä. eingesetzt.

Um zu verhindern, daß aufgrund der hohen an der Austrittsöffnung **(115)** der Sonde **(1)** möglichen Temperaturen sich der Kunststoff des Schutzschlauches **(111)** verflüssigt und somit das Operationsgebiet verunreinigt, kann die Austrittsöffnung **(115)** mit einem hitzebeständigen Material vorzugsweise aus Keramik **(117)** überzogen sein.

Am handstückseitigen Ende der Sonde **(101)** ist eine Kontaktverbindung **(104)** vorgesehen, bei der der ein Innengewinde aufweisende Sondenkopf **(116)** elektrisch leitend durch Schweißen, Löten oder ein anderes zweckmäßiges Verfahren mit der Sonde verbunden ist.
Mittels einer Schraubverbindung **(114)** wird ein gas- und HF-dichter Übergang von dem Adapter **(106)** zum Sondenkopf **(116)** hergestellt. Dieser Adapter **(106)** wird in eine entsprechende Öffnung eines hier nicht abgebildeten Handstückes eingesetzt. Hierbei ist natürlich eine dichte und den elektrischen Kontakt nicht unterbrechende Verbindung zum Handstück sicherzustellen. Diese Verbindung kann durch Klemmen, Pressen, Schrauben etc, erreicht werden.

Der Gas-Strom **(108)** wird durch das Zuführrohr **(105)** des Kupplungsteils **(107)** geleitet. Damit weder Gas an nicht gewollter Stelle austritt, noch es zu hochfrequenten Durchschlägen kommt, wird der Verbindungsbreich durch unterschiedliche Isolierungen vorzugsweise aus Schrumpfschlauch isoliert.

So wird nach dem Verbinden von Sondenrohr **(112)** und Sondenkopf **(116)** dieser Bereich mit der Isolierung **(110)** überzogen. Diese Isolierung überzieht den Sondenkopf **(116)** vollständig und dichtet gegen den Innenbereich der rohrförmigen Verlängerung **(118)** des Kupplungsteiles **(107)** ab. Durch das Aufziehen einer Zusatzisolierung **(103)** erfolgt eine zweite Abdichtung gegen die rohrförmige Verlängerung **(118)**.

Um zu verhindern, daß sich die Schraubverbindung **(114)** während des Einsatzes des Gerätes löst und dann in diesem Arzt und Patienten gefährdenden Bereich eine Undichtigkeit entsteht, wird der Gesamtbereich mittels eines weiteren Schrumpfschlauches **(109)** gesichert.

Der Adapter **(106)** der Sonde **(101)** ist vorzugsweise so gestaltet, daß dieser sowohl an ein Handstück eines Mikro-Elektro-Schutzgasschweißgerätes, als auch an Griffstücke unterschiedlicher Manipulatoren bezw. optischer Geräte angeschlossen werden kann.
Wird die erfindungsgemäße Sonde **(101)** zum Koagulieren eingesetzt, wird nach dem Verbinden mit dem Handstück **(1)** des Mikro-Elektro-Schutzgas-Schweißgerätes die Austrittsöffnung **(115)** für den Plasmastrahl **(113)** der Sonde **(101)** an das zu koagulierende Gewebe herangeführt und nach Öffnen der Gaszufuhr eine hochfrequente Wechselspannung angelegt, die den zum Koagulieren notwendigen Lichtbogen auslöst. Um ein sicheres "Zünden" des Lichtbogens zu gewährleisten, ist für einen guten Kontakt zwischen Gegenpolplatte **(2,102)** und dem Patienten zu sorgen. Die Austrittsöffnung **(115)** der Sonde **(101)** ist vorzugsweise mit einem keramischen Überzug **(117)** versehen, damit nicht wegen der hohen möglichen Temperaturen Kunststoff schmilzt, auf das Operationsfeld tropft und dieses verunreinigt.

Alle Verbindungen zum Handstück **1** -wie Schläuche und Kabel- sind lösbar gestaltet, so daß sich nach Abschluß eines Eingriffs das erfindungsgemäße Handstück **1** und/oder die erfindungsgemäße endoskopische Sonde **(101)** mehrfach im Naßdampf sterilisieren läßt.
- 01 -: Handstück
- 02 -: Griffbereich
- 03 -: Transducer
- 04 -: Gehäusevorderteil
- 05 -: Spülmittelzuleitung
- 06 -: Absaugleitung
- 07 -: Sonotrodenspitze
- 08 -: Sonotrode
- 09 -: Sonotrodenaufnahme
- 10 -: Mündungsbereich
- 11 -: HF-Generator
- 12 -: Abzweigstück
- 13 -: HF - Zuleitung
- 14 -: Ultraschallgenerator
- 15 -: Griffhülse
- 16 -: Anschluß f. Spülmittelleitung
- 17 -: Anschluß für Absaugleitung
- 18 -: Gegenpolplatte
- 19 -: Gaszuführung
- 20 -: HF - Anschluß
- 21 -: Plasmastrahl
- 22 -: Zuleitungen
- 23 -: Ultraschallanschlüsse
- 24 -: Adapter für Gasführungsleitung
- 25 -: Verbindungsteil
- 101 -: endoskopische Sonde
- 102 -: Gegenpolplatte
- 103 -: Zusatzisolierung
- 104 -: Kontaktverbindung
- 105 -: Zuführrohr
- 106 -: Adapter
- 107 -: Kupplungsteil
- 108 -: Gasstrom
- 109 -: Sicherungsschlauch
- 110 -: Isolierung
- 111 -: Leitrohr
- 112 -: Schutzschlauch
- 113 -: Plasmastrahl
- 114 -: Schraubverbindung
- 115 -: Austrittsöffnung
- 116 -: Sondenkopf
- 117 -: keramischer Überzug
- 118 -: rohrförmige Verlängerung

## Patentansprüche

1. Diathermiehandstück für den Einsatz bei chirurgischen Eingriffen insbesondere bei lapraskopischen Ultraschalldissektionen, bei denen eingriffsbedingt auftretende Blutungen durch Verschweißen von Blutgefäßen gestoppt werden müssen, **dadurch gekennzeichnet, daß** im zentralen Bereich des Handstückes **1** eine Gaszuführung **19** angeordnet ist und daß die als Gaszuleitung **19** und Absaugkanal **6** dienende Sonotrode **8** elektrisch leitend ausgestaltet ist.

2. Diathermiehandstück für den Einsatz bei chirurgischen Eingriffen nach Anspruch 1, **dadurch gekennzeichnet, daß** der Spülschlauch **5** vor der Sonotrodenspitze endet, damit Verwirbelungen im Bereich des Plasmastrahles **21** verhindert werden.

3. Diathermiehandstück für den Einsatz bei chirurgischen Eingriffen nach Anspruch 1 und/oder 2, **dadurch gekennzeichnet, daß** die Austrittsöffnung der Sonotrode **8** einen geringeren Innendurchmesser aufweist, als das Gasführungsrohr **19** selbst.

4. Diathermie-Handstück für den Einsatz bei chirurgischen Eingriffen nach Anspruch 1, **dadurch gekennzeichnet, daß** Innen- und Stirnbereich der Sonotrodenspitze **7** mit einem keramischen Überzug versehen sind.

5. Diathermiehandstück für den Einsatz bei chirurgischen Eingriffen nach Anspruch 1, **dadurch gekennzeichnet, daß** alle Versorgungsleitungen lösbar mit dem Handstück **1** verbunden sind.

6. Diathermiehandstück für den Einsatz bei chirurgischen Eingriffen nach einem oder mehreren der vorhergehenden Ansprüche, **dadurch gekennzeichnet, daß** die Gaszuführung separat, d.h. an Transducer **3** und Verbindungsteil **25** vorbei zur Sonotrode **8** geleitet wird.

7. Endoskopische Sonde zur Befestigung an einem Diathermiehandstück für den Einsatz bei endoskopischen Eingriffen, bei denen eingriffsbedingt auftretende Blutungen durch Verschweißen der Gefäße gestoppt werden müssen, **dadurch gekennzeichnet, daß** die endoskopische Sonde **(101)** aus einem mehrschichtig aufgebauten - im Innenbereich elektrisch leitenden- flexiblen Rohr besteht, welches von einem isolierenden und insbesondere gasundurchlässigen Material **(111)** umgeben und mit einem Kupplungsteil **(107)** ausgestattet ist.

8. Endoskopische Sonde nach Anspruch 7, **dadurch gekennzeichnet, daß** der elektrisch leitende Bereich eine Metallspirale ist.

9. Endoskopische Sonde nach Anspruch 7, **dadurch gekennzeichnet, daß** der elektrisch leitende Bereich ein Metallgeflecht ist.

10. Endoskopische Sonde nach einem oder mehreren der vorhergehenden Ansprüche, **dadurch gekennzeichnet, daß** der Bereich der Austrittsöffnung **(115)** mit einem keramischen Überzug **(117)** versehen ist.

11. Endoskopische Sonde nach Anspruch 7, **dadurch gekennzeichnet, daß** ein Kupplungsteil **(107)** mit einem Adapter **(106)** zur Verbindung der Sonde **(101)** mit dem Diathermiehandstück **(1)** oder einem separaten Handstück eines Mikro-Elektro-Schutzgas-Schweißgerätes vorgesehen ist.

12. Endoskopische Sonde nach Anspruch 11, **dadurch gekennzeichnet, daß** das Kupplungsteil **(107)** mit dem Sondenkopf **(116)** lösbar verbunden ist.

13. Endoskopische Sonde nach einem oder mehreren der vorhergehenden Ansprüche, **dadurch gekennzeicnet, daß** der Verbindungsbereich gas- und HF-dicht isoliert ist.

14. Endoskopische Sonde nach Anspruch 13, **dadurch gekennzeichnet, daß** als Isoliermaterial Schrumpfschläuche eingesetzt werden.
